# EUROPEAN PATENT APPLICATION

(11) **EP 4 473 991 A1**
(43) Date of publication of application: **11.12.2024**
(21) Application number: 23747178.4
(22) Date of filing: 31.01.2023
(51) Int. Cl.: A61M 25/00, A61M 25/06, A61M 39/06

(54) **NEEDLE ASSEMBLY**

(30) Priority: 31.01.2022 JP 2022013478
(71) Applicant: Nipro Corporation, Settsu-shi, Osaka 566-8510 (JP)
(72) Inventor: NAKAGAMI, Hiroyuki, Osaka-shi, Osaka 531-8510 (JP); SAKAMOTO, Shingo, Osaka-shi, Osaka 531-8510 (JP); UENISHI, Masayuki, Osaka-shi, Osaka 531-8510 (JP)
(74) Representative: Slingsby Partners LLP
(86) International application number: PCT/JP2023/003094
(87) International publication number: WO 2023/145968

(57) **Abstract**

A needle assembly 10 including: an inner needle 16 with an inner needle hub 18 at its proximal side; a hollow outer needle 58 with an outer needle hub 60 at its proximal side; a needle tip protector 22 protecting a needle tip 20 of the inner needle 16 removed from the outer needle 58; a valve body 80 blocking a lumen of the outer needle hub 60; a pusher 84 pushing a cut 86 of the valve body 80 open by moving toward the needle tip 20 of the inner needle 16 so as to release blockage of the outer needle hub 60 by the valve body 80, the pusher 84 including a push-in piece 108 extending toward a proximal end beside the needle tip protector 22; and a tubular pusher guide 82 guiding movement of the pusher 84 toward the needle tip 20, the pusher guide 82 including an insertion area 96 which allows insertion of the push-in piece 108 therein.

## Description

### TECHNICAL FIELD

The present invention relates to a needle assembly used for blood collection, fluid transfusion (including blood transfusion), and the like.

### BACKGROUND ART

Conventionally, a needle assembly has been used in blood collection, fluid transfusion and the like. The needle assembly has a structure wherein an inner needle with an inner needle hub attached to its proximal end is inserted through a hollow outer needle with an outer needle hub attached to its proximal end. After the inner needle and the outer needle puncture a blood vessel, the inner needle is extracted from the outer needle, so that the outer needle and the outer needle hub are left in a state of puncturing the vessel. Then, an external circuit such as a syringe or a tube is connected to the indwelling outer needle hub to perform blood collection, fluid transfusion, or the like.

As disclosed in International Publication No. WO 2013/051242 (Patent Document 1), there was proposed the needle assembly structured including a valve body that can open and close the lumen of the outer needle hub. In the needle assembly equipped with this valve body, when the inner needle is removed, the valve body closes the lumen of the outer needle hub to suppress blood leakage. Additionally, when the external circuit is connected to the indwelling outer needle hub, a pusher is guided by a pusher guide and moves toward the distal side to push the valve body open, thereby switching the lumen of the outer needle hub from an obstruction state to a communication state.

Besides, as disclosed in International Publication No. WO 2004/004819 (Patent Document 2), there was also proposed a structure with a needle tip protector that protects the tip of the inner needle when removing the inner needle to prevent accidental puncture, etc. In Patent Document 2, pushers are provided on both sides of the needle tip protector to extend toward the proximal end. This shortens the axial length of the outer needle hub that houses the pushers and the needle tip protector, while enabling the pushers to be pushed in from the proximal side to the distal side when opening the valve.

### BACKGROUND ART DOCUMENTS

### PATENT DOCUMENTS

Patent Document 1: WO 2013/051242
Patent Document 2: WO 2004/004819

### SUMMARY OF THE INVENTION

### PROBLEM THE INVENTION ATTEMPTS TO SOLVE

However, in the structure of Patent Document 1, the pusher moves toward the distal side relative to the pusher guide. Therefore, a gap allowing the movement stroke is required between the pusher and the pusher guide so that the pusher's movement toward the distal end is not hindered by interference with the pusher guide. As a result, there is a risk that the needle assembly (the outer needle hub) may become larger in the axial direction.

Moreover, in the case of Patent Document 2 wherein the pushers extend toward the proximal end on the both sides of the needle tip protector, if the pushers rotate relative to the outer needle hub, the parts of the pushers extending toward the proximal end may interfere with the needle tip protector, affecting deformation and movement of the needle tip protector, which might increase resistance when the inner needle is removed.

The present invention has been developed in view of the above-described matters as the background, and it is an object of the present invention to provide a needle assembly with a novel structure which is able to solve at least one of the problems of the conventional needle assemblies described above.

### MEANS FOR SOLVING THE PROBLEM

Hereinafter, preferred embodiments for grasping the present invention will be described. However, each preferred embodiment described below is exemplary and can be appropriately combined with each other. Besides, a plurality of elements described in each preferred embodiment can be recognized and adopted as independently as possible, or can also be appropriately combined with any element described in other preferred embodiments. By so doing, in the present invention, various other preferred embodiments can be realized without being limited to those described below.

A first preferred embodiment provides a needle assembly comprising: an inner needle with an inner needle hub at its proximal side; a hollow outer needle with an outer needle hub at its proximal side; a needle tip protector protecting a needle tip of the inner needle removed from the outer needle; a valve body blocking a lumen of the outer needle hub; a pusher pushing a cut of the valve body open by moving toward the needle tip of the inner needle so as to release blockage of the outer needle hub by the valve body, the pusher including a push-in piece extending toward a proximal end beside the needle tip protector; and a tubular pusher guide guiding movement of the pusher toward the needle tip, the pusher guide including an insertion area which allows insertion of the push-in piece therein.

With the needle assembly structured according to the present preferred embodiment, the push-in piece of the pusher extends toward the proximal end beside the needle tip protector. Therefore, it is possible to set the proximal end of the pusher at a position wherein the pusher is easily pushed in to the distal side through the proximal end opening of the outer needle hub, while securing a space for housing the needle tip protector in the outer needle hub without requiring a larger size of the outer needle hub.

In addition, the pusher guide is provided with the insertion area into which the push-in piece of the pusher is inserted, when the pusher is pushed in to the distal side to open the valve body. Thus, it is possible to prevent interference between the push-in piece and the pusher guide from hampering the movement of the pusher to the distal side. Therefore, the travel stroke of the pusher to the distal side can be secured without the need to increase the size of the outer needle hub.

A second preferred embodiment provides the needle assembly according to the first preferred embodiment, wherein a notch penetrating a circumferential wall of the tubular pusher guide, while opening an inside to an outside, is provided as the insertion area.

With the needle assembly structured according to the present preferred embodiment, the push-in piece of the pusher is inserted into the notch constituting the insertion area, in a state wherein the outer peripheral side of the push-in piece of the pusher is exposed without being covered by the pusher guide. This facilitates reduction in the diameter of the pusher guide.

In this preferred embodiment, it is possible to provide a single insertion area, or a plurality of insertion areas. When the plurality of insertion areas are provided, it will do as long as at least one of the plurality of insertion areas is the notch. The other insertion areas can be of different structures from the notch, such as groove-shaped.

A third preferred embodiment provides the needle assembly according to the first or second preferred embodiment, further comprising a protector rotation restriction part provided at a distal end of the inner needle hub and inserted between the needle tip protector and the outer needle hub so as to restrict rotation of the needle tip protector relative to the inner needle hub by contacting the needle tip protector.

In the needle assembly constructed according to the present preferred embodiment, when the outer needle hub and the inner needle hub are properly positioned in the circumferential direction, it is possible to properly and easily set the circumferential orientation of the needle tip protector relative to the inner needle hub and the outer needle hub based on the action of the protector rotation restriction part. Furthermore, it is possible to compactly configure the protector rotation restriction part that restricts the rotation of the needle tip protector relative to the inner needle hub, in the outer needle hub.

A fourth preferred embodiment provides the needle assembly comprising: an inner needle with an inner needle hub at its proximal side; a hollow outer needle with an outer needle hub at its proximal side; a needle tip protector protecting a needle tip of the inner needle removed from the outer needle; a valve body blocking a lumen of the outer needle hub; a pusher pushing a cut of the valve body open by moving toward the needle tip so as to release blockage of the outer needle hub by the valve body, the pusher including a push-in piece extending toward a proximal end beside the needle tip protector; a tubular pusher guide attached to the outer needle hub to guide movement of the pusher toward the needle tip; and a rotation limiting mechanism limiting rotation of the pusher relative to the pusher guide assembled and retained in the outer needle hub.

With the needle assembly structured according to the present preferred embodiment, the rotation of the pusher relative to the pusher guide attached to the outer needle hub is limited by the rotation limiting mechanism, which can prevent the pusher and the needle tip protector from easily interfering (contacting) with each other through relative rotation. This prevents unintended displacement of the needle tip protector due to the interference of the pusher, which avoids an increase in resistance on removal of the inner needle due to the needle tip protector being displaced and pressed against the inner needle, for example.

A fifth preferred embodiment provides the needle assembly according to the fourth preferred embodiment, wherein the rotation limiting mechanism is constituted by a contact section between the pusher and the pusher guide so as to limit relative rotation of the pusher and the pusher guide in a circumferential direction by mutual contact.

With the needle assembly structured according to the present preferred embodiment, the rotation limiting mechanism can be easily realized without requiring special components or complicated mechanisms.

In the fifth preferred embodiment, for example, the outer peripheral surface of the tapered distal end section of the pusher may be provided with a first rotation regulating surface whose curvature is smaller than that of other portions in the circumferential direction, while the inner surface of the pusher guide may be provided with a second rotation regulating surface to be overlapped on the first rotation regulating surface. The first rotation regulating surface and the second rotation regulating surface may be overlapped on each other to form the contact section.

With the needle assembly structured according to the present preferred embodiment, the rotation limiting mechanism can be easily realized by a simple structure wherein the first rotation regulating surface and the second rotation regulating surface are overlapped on each other. Moreover, high strength and durability can be easily secured by configuring the contact section of mutual contact with the first rotation regulating surface and the second rotation regulating surface. Furthermore, the first and second rotation regulating surfaces can be easily molded when molding the pusher and pusher guide, and the mold structure can be simplified.

A sixth preferred embodiment provides the needle assembly according to the fifth preferred embodiment, wherein a proximal end of a tapered distal end section of the pusher is provided with a step surface, and a cross section of the proximal end of the tapered distal end section of the pusher is not a true circle, and the pusher guide is provided with a detent projection to prevent removal of the pusher by contacting the step surface, and the contact section is constituted by a projecting distal end surface of the detent projection of the pusher guide and an outer peripheral surface of the pusher.

With the needle assembly structured according to the present preferred embodiment, the pusher is held in an appropriate position relative to the pusher guide because the pusher is prevented from slipping out of the pusher guide to the proximal side by utilizing the detent projection of the pusher guide, which constitutes the contact section between the outer peripheral surface of the pusher and the pusher guide. Also, since the projecting distal end surface of the detent projection and the outer peripheral surface of the pusher constitute the contact section, the contact section is readily set in close proximity to the pusher and the pusher guide, thereby making it possible to efficiently limit the rotation of the pusher relative to the pusher guide.

A seventh preferred embodiment provides the needle assembly according to any one of the first through sixth preferred embodiments, wherein an inner diameter dimension of the outer needle hub is larger in a proximal portion than in a distal portion, and a part of the push-in piece of the pusher that is inserted into the proximal portion of the outer needle hub has a large-diameter thick-walled portion with a large outer diameter dimension and a thick wall.

With the needle assembly structured according to the present preferred embodiment, since the proximal end of the push-in piece is the large-diameter thick-walled portion, a large contact area with a male connector or the like can be obtained when the push-in piece is pushed in to the distal side by the male connector or the like connected to the outer needle hub, for example. Additionally, by inserting the large-diameter thick-walled portion of the push-in piece into the proximal portion of the outer needle hub with a larger inner diameter dimension, the gap between the outer needle hub and the push-in piece can be prevented from becoming excessively large.

An eighth preferred embodiment provides the needle assembly according to any one of the first through seventh preferred embodiments, wherein a radially inner edge of a proximal end of the pusher guide is provided with an edge widening part that slopes radially outward as it goes toward the proximal end.

With the needle assembly structured according to the present preferred embodiment, when the pusher is inserted into the pusher guide from the proximal side, the pusher is guided by the contact with the edge widening part, and thus the pusher can be easily arranged in a suitable position relative to the pusher guide.

A ninth preferred embodiment provides the needle assembly comprising: an inner needle with an inner needle hub at its proximal side; a hollow outer needle with an outer needle hub at its proximal side; a needle tip protector being positioned in the outer needle hub and protecting a needle tip of the inner needle removed from the outer needle; and a protector rotation restriction part provided in the inner needle hub so as to restrict rotation of the needle tip protector relative to the inner needle hub.

With the needle assembly structured according to the present preferred embodiment, since the protector rotation restriction part restricts the rotation of the needle tip protector relative to the inner needle hub, if the outer needle hub and the inner needle hub are oriented appropriately in the circumferential direction, the circumferential orientation of the needle tip protector relative to the outer needle hub can be easily set to the appropriate orientation. The protector rotation restriction part may have a shape of protrusion provided at the distal end of the inner needle hub and a simple structure that restricts the rotation of the needle tip protector by being inserted between the needle tip protector and the outer needle hub, for example.

A tenth preferred embodiment provides the needle assembly comprising: an inner needle with an inner needle hub at its proximal side; a hollow outer needle with an outer needle hub at its proximal side; a needle tip protector protecting a needle tip of the inner needle removed from the outer needle; a valve body blocking a lumen of the outer needle hub; a pusher pushing a cut of the valve body open by moving toward the needle tip of the inner needle so as to release blockage of the outer needle hub by the valve body, the pusher including a push-in piece extending toward a proximal end beside the needle tip protector; and a valve holding member holding the valve body in an attached state to the outer needle hub, the valve holding member including an insertion area which allows insertion of the push-in piece therein.

With the needle assembly structured according to the present preferred embodiment, the push-in piece of the pusher extends toward the proximal end beside the needle tip protector. Therefore, it is possible to set the proximal end of the pusher at a position wherein the pusher is easily pushed in to the distal side through the proximal end opening of the outer needle hub, while securing a space for housing the needle tip protector in the outer needle hub without requiring a larger size of the outer needle hub.

In addition, the push-in piece of the pusher is inserted into the insertion area provided in the valve holding member holding the valve body, when the pusher is pushed in to the distal side to open the valve body. Thus, it is possible to prevent interference between the push-in piece and the valve holding member from hampering the movement of the pusher to the distal side. Therefore, the travel stroke of the pusher to the distal side can be secured without the need to increase the size of the outer needle hub.

An eleventh preferred embodiment provides the needle assembly comprising: an inner needle with an inner needle hub at its proximal side; a hollow outer needle with an outer needle hub at its proximal side; a valve body blocking a lumen of the outer needle hub; a pusher pushing a cut of the valve body open by moving toward a needle tip of the inner needle so as to release blockage of the outer needle hub by the valve body; and a tubular pusher guide guiding movement of the pusher toward the needle tip, the pusher guide including an insertion area into which the pusher is inserted therein partially in a circumferential direction by the movement of the pusher toward the needle tip.

With the needle assembly structured according to the present preferred embodiment, the pusher is inserted into the insertion area of the pusher guide partially in the circumferential direction, when the pusher is pushed in to the distal side to open the valve body. Thus, it is possible to prevent interference between the pusher and the push-in piece from hampering the movement of the pusher to the distal side. Therefore, the travel stroke of the pusher to the distal side can be secured without the need to increase the size of the outer needle hub.

### EFFECT OF THE INVENTION

According to the present invention, it is possible to prevent the needle assembly (the outer needle hub) from becoming larger in the axial direction while securing the travel stroke of the pusher to the distal side and/or avoiding unintended deformation or movement of the needle tip protector.

### BRIEF DESCRIPTION OF THE DRAWINGS

FIG. 1 is a cross-section view showing a needle assembly as a first practical embodiment of the present invention, taken along line 1-1 of FIG. 2.
FIG. 2 is a cross-section view taken along line 2-2 of FIG. 1.
FIG. 3 is a cross-section view taken along line 3-3 of FIG. 1.
FIG. 4 is a cross-section view taken along line 4-4 of FIG. 1.
FIG. 5 is a perspective view of a needle tip protector constituting the needle assembly shown in FIG. 1.
FIG. 6 is a perspective view showing the needle tip protector shown in FIG. 5 from another angle.
FIG. 7 is a perspective view showing a pusher guide constituting the needle assembly shown in FIG. 1.
FIG. 8 is a perspective view showing the pusher guide shown in FIG. 7 from another angle.
FIG. 9 is a perspective view showing a pusher constituting the needle assembly shown in FIG. 1.
FIG. 10 is a perspective view showing the pusher shown in FIG. 9 from another angle.
FIG. 11 is a cross-section view showing a state wherein an inner needle unit is withdrawn from an outer needle unit in the needle assembly shown in FIG. 1, taken along line 11-11 of FIG. 12.
FIG. 12 is a cross-section view taken along line 12-12 of FIG. 11.
FIG. 13 is a cross-section view showing a state wherein an external circuit is connected to the outer needle unit shown in FIG. 11, taken along line 13-13 of FIG. 14.
FIG. 14 is a cross-section view taken along line 14-14 of FIG. 13.

### EMBODIMENTS FOR CARRYING OUT THE INVENTION

Practical embodiments of the present invention will be described with reference to the drawings.

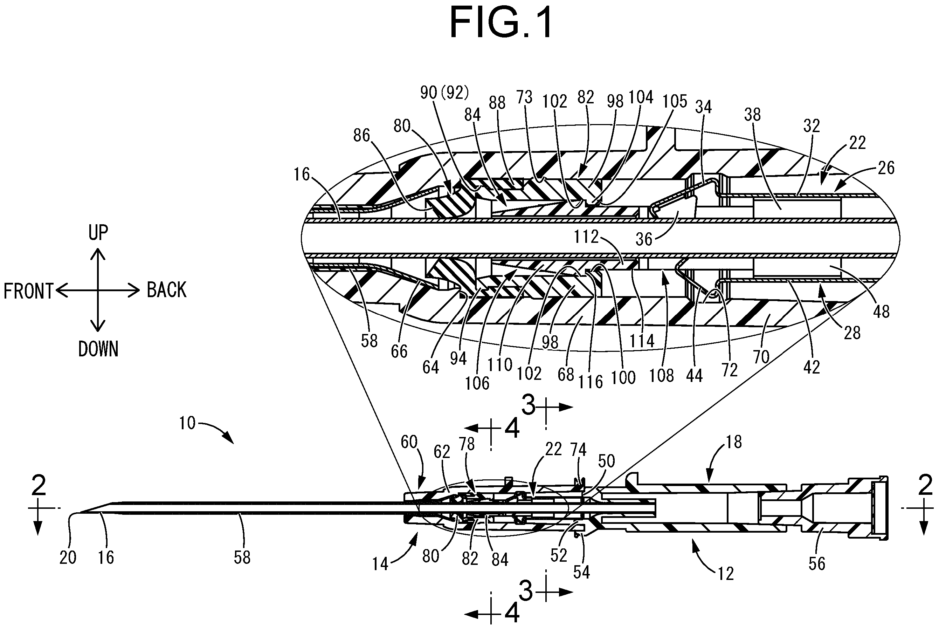

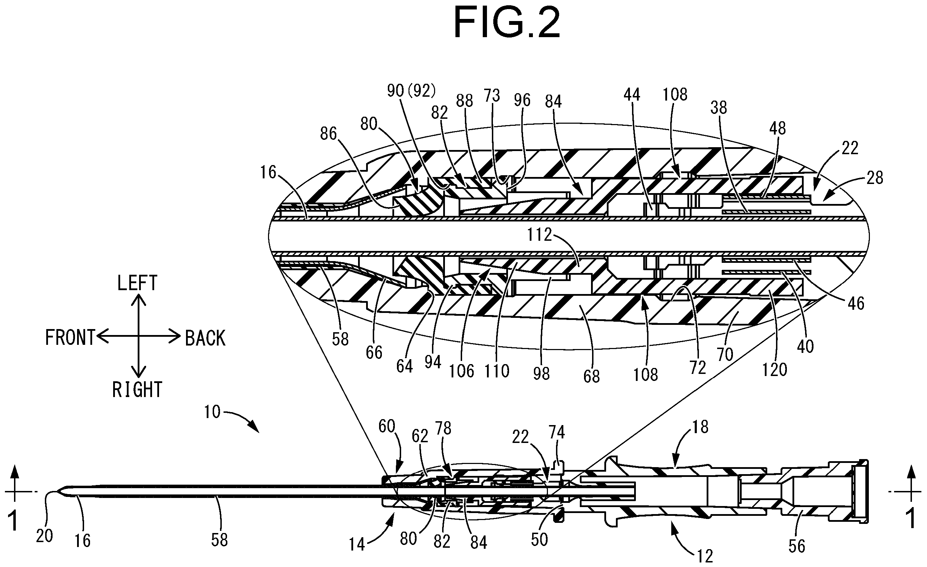

FIGS. 1 to 4 show a needle assembly 10 as a first practical embodiment of this invention. The needle assembly 10 has a structure wherein an inner needle unit 12 and an outer needle unit 14 are detachably connected to each other. In the following explanation, the front-back direction (the axial direction) is, in principle, the left-right direction in FIG. 1, which is the needle axis direction of an inner needle 16 and an outer needle 58 described below. Besides, in principle, the distal side means the left side in FIG. 1, which is the side of a needle tip 20 of the inner needle 16 described below, while the proximal side means the right side in FIG. 1, which is the side grasped and operated by the user. In principle, the up-down direction means the up-down direction in FIG. 1, and the left-right direction means the left-right direction in FIG. 2.

The inner needle unit 12 has a structure wherein an inner needle hub 18 is fixed to the proximal side of the inner needle 16. The inner needle 16 is formed of a known material such as stainless steel, aluminum, titanium, or an alloy of some of them, and in this practical embodiment, it is a hollow needle. The inner needle 16 may be a solid needle. The distal end of the inner needle 16 is the sharp needle tip 20.

A needle tip protector 22 is attached to the inner needle 16. The needle tip protector 22 is formed of a metallic material such as medical stainless steel, for example. In this practical embodiment, components of the needle tip protector 22 described below are formed integrally, and the needle tip protector 22 can be easily manufactured, for example, by press working on a metal blank plate.

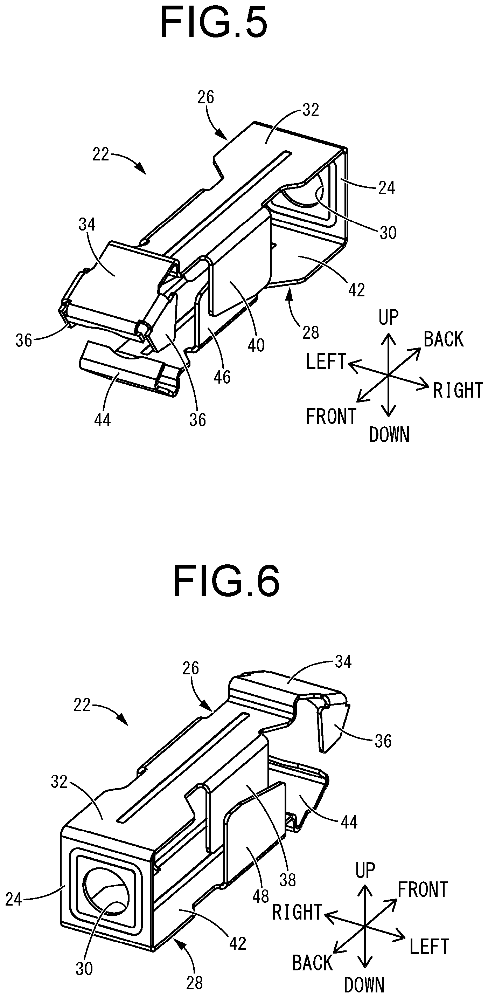

As shown in FIGS. 5 and 6, the needle tip protector 22 has a structure wherein a first side plate part 26 and a second side plate part 28 extend out toward the needle tip 20 from the top and bottom edges of a bottom part 24 located at the proximal end (opposite the needle tip 20). In FIGS. 5 and 6, the needle tip protector 22 is shown in the pre-activated state (before needle tip protection) for illustrative purposes, but the needle tip protector 22 may be in the post-activated state (during needle tip protection) in isolation and it may change into the pre-activated state shown in FIGS. 5 and 6 by attachment to the inner needle 16.

As shown in FIG. 6, the bottom part 24 has an approximately rectangular flat plate shape and is provided with an insertion hole 30 that penetrates it in the plate thickness direction. The first side plate part 26 and the second side plate part 28 are formed integrally at a pair of opposite sides of the bottom part 24 and extend to one side (the distal side) in the plate thickness direction of the bottom part 24. The first side plate part 26 and the second side plate part 28 in this practical embodiment are arranged on the upper and lower sides of the inner needle 16 and opposed to each other and do not extend in an intersecting manner.

The first side plate part 26 has an approximately rectangular plate shape as a whole and is elongated in the front-back direction, which is the direction of extension from the bottom part 24. The first side plate part 26 has a first main body 32 in a flat plate shape connected to the bottom part 24, and also has a first needle tip protection part 34 on the distal side of the first main body 32. The first needle tip protection part 34 extends from the distal end of the first main body 32 to the opposite side to the second side plate part 28, and bends to protrude to the distal side while inclining toward the second side plate part 28, and the distal end protrudes toward the bottom part 24 while inclining toward the second side plate part 28. On both sides of the first needle tip protection part 34 in the width direction, there are provided a pair of lateral guarding pieces 36, 36 that expand substantially orthogonally to the width direction. The first side plate part 26 has a first lateral cover piece 38 extending from one edge of the first main body 32 in the width direction toward the second side plate part 28, and a first projection piece 40 extending from the other edge of the first main body 32 in the width direction toward the second side plate part 28. The first lateral cover piece 38 and the first projection piece 40 are provided on both sides of the first main body 32 in the width direction, and are mutually spaced apart, facing each other in the width direction.

The second side plate part 28 has an approximately rectangular plate shape as a whole and is elongated in the front-back direction, which is the direction of extension from the bottom part 24. The second side plate part 28 has a second main body 42 in a flat plate shape connected to the bottom part 24, and also has a second needle tip protection part 44 on the distal side of the second main body 42. The second needle tip protection part 44 extends from the distal end of the second main body 42 to the opposite side to the first side plate part 26, and bends to protrude to the distal side while inclining toward the first side plate part 26, and the distal end protrudes toward the bottom part 24 while inclining toward the first side plate part 26. The second side plate part 28 has a second lateral cover piece 46 extending from one edge of the second main body 42 in the width direction toward the first side plate part 26, and a second projection piece 48 extending from the other edge of the second main body 42 in the width direction toward the first side plate part 26. The second lateral cover piece 46 and the second projection piece 48 are provided on both sides of the second main body 42 in the width direction, and are mutually spaced apart, facing each other in the width direction.

The first lateral cover piece 38 and the second projection piece 48 are provided on the same side edges in the width direction of the first side plate part 26 and the second side plate part 28, and they overlap each other in the width direction when being attached to the inner needle 16 before needle tip protection, and the first lateral cover piece 38 is spaced inside the second projection piece 48 in the width direction. The second lateral cover piece 46 and the first projection piece 40 are provided on the same side edges in the width direction of the first side plate part 26 and the second side plate part 28, and they overlap each other in the width direction when being attached to the inner needle 16 before needle tip protection, and the second lateral cover piece 46 is spaced inside the first projection piece 40 in the width direction. The first lateral cover piece 38 and the second lateral cover piece 46 are provided mutually opposite in the width direction and separated from each other while facing each other in the width direction. The first projection piece 40 and the second projection piece 48 are provided mutually opposite in the width direction and separated from each other while facing each other in the width direction, and located on both outsides in the width direction relative to the first and second lateral cover pieces 38, 46.

The needle tip protector 22 is attached to the inner needle 16 as shown in FIGS. 1 and 2. Specifically, the inner needle 16 is inserted into the insertion hole 30 formed in the bottom part 24 of the needle tip protector 22 and between the first needle tip protection part 34 and the second needle tip protection part 44. By so doing, the inner needle 16 is arranged through between the first side plate part 26 and the second side plate part 28 of the needle tip protector 22 which face each other, so that the needle tip protector 22 is externally fitted and attached to the inner needle 16. The inner needle 16 is located between the first lateral cover piece 38 and the second lateral cover piece 46 of the needle tip protector 22 which face each other. In a part in the needle axis direction, the inner needle 16 is surrounded by the first and second side plate parts 26, 28 and the first and second lateral cover pieces 38, 46. The needle tip protector 22 is movable in the needle axis direction relative to the inner needle 16, from a position wherein it is adjacent to the inner needle hub 18 to a position wherein it covers the needle tip 20 of the inner needle 16.

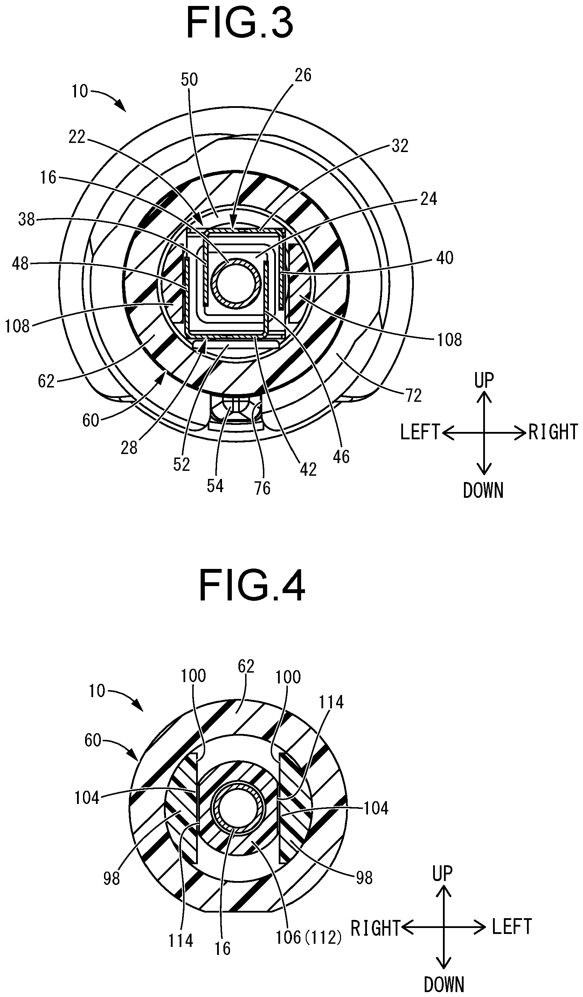

The inner needle hub 18 is a generally cylindrical member formed of a hard synthetic resin. The distal end of the inner needle hub 18 has a cylindrical connection section 50 that can be inserted into the proximal end opening of an outer needle hub 60 (described below). The connection section 50 has a protector rotation restriction part 52 with a longer protruding length to the distal side in part in the circumferential direction. The needle tip protector 22 is located on the distal side of a portion of the connection section 50 outside the protector rotation restriction part 52. The protector rotation restriction part 52 extends to the lower side of the needle tip protector 22 and is overlapped on the proximal end of the needle tip protector 22 (the proximal end of the second side plate part 28) from outside in the up-down direction, as also shown in FIG. 3. As a result, the amount of rotation of the needle tip protector 22 relative to the inner needle hub 18 is restricted by contact between the second side plate part 28 and the protector rotation restriction part 52. In the distal end of the inner needle hub 18, a circumferential positioning part 54 is provided radially outside the protector rotation restriction part 52 to face the protector rotation restriction part 52.

A filter cap 56 is attached to the proximal end opening of the inner needle hub 18, as shown in FIGS. 1 and 2. The filter cap 56 is tubular and the proximal end opening of the lumen thereof is covered by a gas-permeable filter that blocks passage of blood and allows passage of air. This allows blood to flow from the distal end of the inner needle 16 into the lumen of the inner needle hub 18, and the filter cap 56 prevents blood that has flowed into the lumen of the inner needle hub 18 from leaking to the outside.

The outer needle unit 14 has a structure wherein the outer needle hub 60 is fixed to the proximal side of the outer needle 58. The outer needle 58 is a hollow needle with an axially penetrating lumen. The outer needle 58 suitably has some flexibility. The outer surface at the distal end of the outer needle 58 may be tapered with a gradually decreasing diameter as it goes toward the distal side, for example, which can decrease resistance during puncture into the patient, thereby reducing pain and the like. The peripheral wall at the distal end of the outer needle 58 may be provided with one through hole or a plurality of through holes, which can improve the efficiency of fluid flow from and into the outer needle 58.

The outer needle hub 60 has a substantially tubular shape extending in the axial direction as a whole, and in this practical embodiment, it has an approximately cylindrical peripheral wall 62. The inner surface of the peripheral wall 62 is provided with a step 64, and the distal side of the step 64 has a smaller diameter than the proximal side thereof. A generally cylindrical swaging pin 66 is fixed to the inner surface of the peripheral wall 62 on the distal side of the step 64, and the proximal end of the outer needle 58 inserted into the peripheral wall 62 is sandwiched between the peripheral wall 62 and the swaging pin 66, and bonded or welded depending on the necessity, and thus the outer needle hub 60 is fixed to the proximal side of the outer needle 58.

In the proximal side of the step 64, the inner diameter dimension of the peripheral wall 62 of the outer needle hub 60 is larger in a proximal portion 70 than in a distal portion 68. The proximal portion 70 of the peripheral wall 62 has a luer taper set on its inner surface, and the inner diameter dimension decreases as it goes toward the distal end. The inner diameter dimension at the distal end of the proximal portion 70 is larger than the maximum inner diameter dimension of the distal portion 68. At the boundary between the distal portion 68 and the proximal portion 70, an annular protector engagement groove 72 is formed opening to the inner surface and extending in the circumferential direction. An annular concave groove 73 extending in the circumferential direction is formed on the inner surface of the distal portion 68.

At the proximal end of the outer needle hub 60, a flanged portion 74 projecting radially outward is provided around the substantially entire circumference. On the radially outer surface of the flanged portion 74, a screw thread is provided, so it is possible to connect a luer-lock syringe, connector, or the like to the outer needle hub 60. In this practical embodiment, a positioning groove 76 extending in the axial direction is provided in a portion of the circumference in the flanged portion 74.

A hemostasis valve mechanism 78 is incorporated inside the outer needle hub 60. The hemostasis valve mechanism 78 has a valve body 80, a pusher guide 82, and a pusher 84. As described below, when the inner needle 16 is extracted, the area on the distal side of the outer needle hub 60 relative to the hemostasis valve mechanism 78 contains the blood that has flowed in through the outer needle 58. Therefore, the peripheral wall 62 of the outer needle hub 60 is transparent at least on the distal side relative to the position at which the hemostasis valve mechanism 78 is arranged, so that the user, etc. can check the flashback from the outside.

The valve body 80 is made of an elastic material such as rubber or elastomer, and has a cut 86 in the center thereof. The outer peripheral end of the distal end surface of the valve body 80 is overlapped on the step 64 provided on the inner surface of the outer needle hub 60, thereby regulating movement toward the distal side. A tubular support piece 88 is integrally formed at the outer peripheral end of the valve body 80, protruding toward the proximal side. A retaining groove 90 is formed on the proximal end surface of the valve body 80, extending in an annular shape along the inner circumference of the support piece 88. The retaining groove 90 is provided with a widened portion 92 that expands to the radially outer side at least in a part in the circumferential direction, partially in the circumferential direction. The valve body 80 is compressed in the radial direction by attachment to the outer needle hub 60, and the inner surfaces of the cut 86 are in close contact with each other and the cut 86 is blocked, so that the lumen of the outer needle hub 60 is liquid-tightly blocked by the valve body 80.

The tubular pusher guide 82 is located at the proximal side of the valve body 80. The pusher guide 82 is inserted in the support piece 88 of the valve body 80, and the support piece 88 is held between the outer needle hub 60 and the pusher guide 82 in the radial direction. The distal end of the pusher guide 82 is inserted in the retaining groove 90 of the valve body 80, and the outer circumference portion of the valve body 80 is clasped between the step 64 of the outer needle hub 60 and the pusher guide 82 in the axial direction. The distal end of the pusher guide 82 inserted in the retaining groove 90 has an outer circumferential projection 94 projecting radially outward, in part in the circumferential direction. By inserting the outer circumferential projection 94 into the widened portion 92 of the retaining groove 90, the valve body 80 and the pusher guide 82 are mutually positioned in the circumferential direction.

The pusher guide 82 is fixed and attached to the outer needle hub 60, in a state wherein the proximal side of the pusher guide 82, relative to the insertion portion into the support piece 88 of the valve body 80, is fitted to the peripheral wall 62 of the outer needle hub 60. As a result, the pusher guide 82 of this practical embodiment is a valve holding member that holds the valve body 80 in the attached state to the outer needle hub 60. In this practical embodiment, the distal end of the portion of the pusher guide 82 fitted to the outer needle hub 60 slightly projects radially outward and has a large diameter. When the pusher guide 82 is assembled to the outer needle hub 60, the large diameter portion of the pusher guide 82 is inserted into the concave groove 73 provided on the inner surface of the outer needle hub 60 and they click on insertion, so that it is possible to easily grasp completion of appropriate arrangement of the pusher guide 82 relative to the outer needle hub 60 in the axial direction by the clicking sensation.

The pusher guide 82 of this practical embodiment is fitted to the inner surface of the outer needle hub 60 and is indirectly pressed against the outer needle hub 60 via the valve body 80, which is an elastic body. The positioning action of the pusher guide 82 relative to the outer needle hub 60 is exerted by the frictional resistance force acting between the pusher guide 82 and the valve body 80 with a large friction coefficient. These prevent the pusher guide 82 from rotating or moving relative to the outer needle hub 60, even if an external force such as a rotational force is exerted on the pusher guide 82 from the pusher 84 described below, when the pusher 84 is pushed to the distal side, for example.

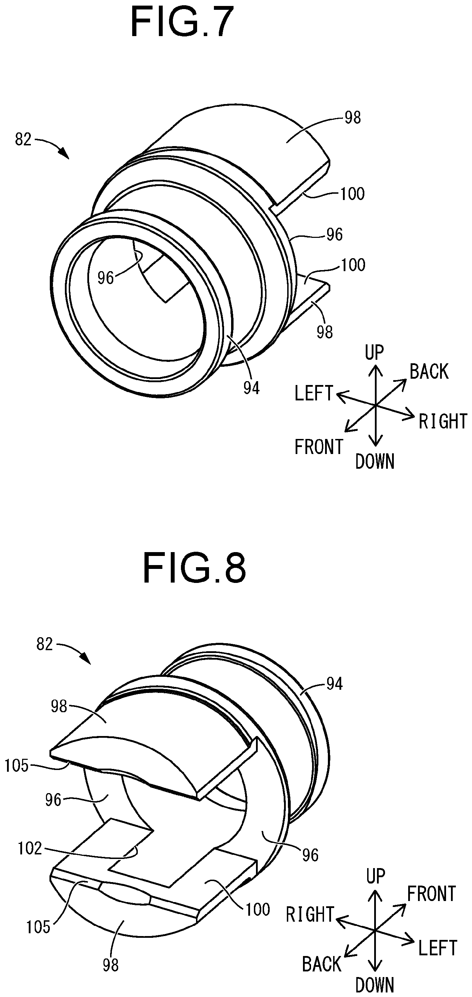

The proximal end of the pusher guide 82 has a pair of notches 96, 96, as shown in FIGS. 7 and 8. The notches 96, 96 as insertion areas are formed on both the left and right sides of the pusher guide 82 and are provided penetrating the proximal end of the pusher guide 82 in the left-right direction. By forming the pair of notches 96, 96, the proximal end of the pusher guide 82 is made into a pair of guide pieces 98, 98 located off the pair of notches 96, 96 in the circumferential direction and between the notches 96, 96 in the circumferential direction. The pair of guide pieces 98, 98 are arranged opposite each other in the up-down direction and are substantially symmetrical in shape. The outer circumferential surface of the guide piece 98 has an arc-shaped cross-section corresponding to the inner surface of the outer needle hub 60, while the inner surface thereof is a guide-side flat surface 100 as a second rotation regulating surface whose curvature is smaller than that of the outer circumferential surface. In this practical embodiment, the guide-side flat surface 100 has a plane shape. For the guide piece 98, the curvature of the inner surface is smaller than that of the outer circumferential surface, so that the guide piece 98 has a thicker wall as it goes toward the center in the circumferential direction.

In the distal ends of the pair of guide pieces 98, 98, groove-shaped recesses 102, 102 are formed extending with an arc-shaped cross-section in the axial direction, while opening to the inner surface thereof. The inner surface of the groove-shaped recess 102 is located on an extension of the inner surface of the distal side relative to the guide piece 98 in the pusher guide 82. The groove-shaped recess 102 does not extend to the proximal end of the guide piece 98. On the proximal side of the guide piece 98 relative to the groove-shaped recess 102, a detent projection 104 is provided that projects inwardly in the facing direction of the pair of guide pieces 98, 98. For the detent projection 104, the surface on its distal end side (the needle tip 20 side) expands in the axis-perpendicular direction, while the surface on its proximal end side is a tapered edge widening part 105 which slopes radially outward as it goes toward the proximal end. As shown in FIG. 8, in the edge widening part 105 of the present practical embodiment, the both left-right side portions are composed of flat surfaces, while the center portion in the left-right direction has an arc-shaped cross section. The projecting distal end surface (the inner end face in the up-down direction) of the detent projection 104 is composed of a portion of the guide-side flat surface 100, and is a plane that expands as substantially orthogonal to the up-down direction.

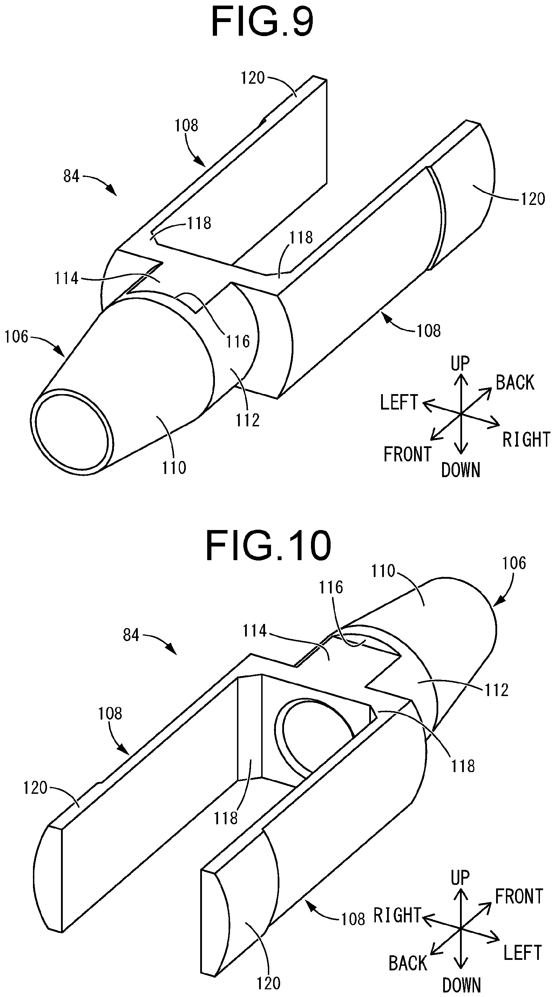

The pusher 84 is inserted in the pusher guide 82. The pusher 84 is made of a hard synthetic resin, for example, and as shown in FIGS. 9 and 10, the distal end of the pusher 84 is a cylindrical insertion tube 106 extending in the needle axis direction and the pusher 84 has a pair of push-in pieces 108, 108 on the proximal side of the insertion tube 106.

The insertion tube 106 has a tapered distal end section 110 with a tapered outer circumferential surface that decreases in diameter as it goes toward the distal end, and a flat proximal end section 112 that extends in the needle axis direction with an almost constant cross-sectional shape. The inner surface of the insertion tube 106 is a cylindrical surface extending in the needle axis direction with an almost constant diameter dimension over the entire length. Accordingly, the insertion tube 106 has a thinner wall as it goes toward the distal end in the tapered distal end section 110.

The flat proximal end section 112 of the pusher 84 has a pair of upper and lower pusher-side flat surfaces 114, 114 on its outer circumferential surface. The pusher-side flat surfaces 114 comprise planar surfaces that spread as substantially orthogonal to the up-down direction, and the flat proximal end section 112 has a flat cross-sectional shape in which the outer dimension in the up-down direction is smaller than the outer dimension in the left-right direction. As a result, the proximal end of the tapered distal end section 110 projects outward in the up-down direction relative to the flat proximal end section 112, on both the upper and lower sides where the pusher-side flat surfaces 114 are formed. By so doing, a step surface 116 is formed at the proximal end of the tapered distal end section 110, to spread in the axis-perpendicular direction at the boundary portion with the flat proximal end section 112. In this practical embodiment, the length dimension of the flat proximal end section 112 is shorter than the length dimension of the tapered distal end section 110. Preferably, the length dimension of the flat proximal end section 112 is within the range of 1/4 to 3/4 of the length dimension of the tapered distal end section 110. Since the inner surface of the flat proximal end section 112 is a cylindrical surface, the radial thickness dimension of the flat proximal end section 112 is smaller in the areas where the pusher-side flat surfaces 114 are formed, than in the other areas.

The pair of push-in pieces 108, 108 are integrally provided at the proximal end side of the pusher 84 relative to the flat proximal end section 112. The push-in pieces 108 are provided partially in the circumferential direction at the proximal end portion of the pusher 84. The left and right outer surfaces of the push-in pieces 108, 108 are curved surfaces having arcuate cross-sections, and the left and right inner surfaces are flat and spread as substantially orthogonal to the left-right direction, so that the push-in pieces 108, 108 have thicker walls as they go toward the center in the up-down direction. The distal end portion of the push-in piece 108 is bent inward in the left-right direction and connected integrally to the proximal end portion of the flat proximal end section 112. In other words, the pair of push-in pieces 108, 108 extend outward in the left-right direction from the proximal end portion of the flat proximal end section 112 and are bent at both the left and right outer ends to extend to the proximal end side. The distal end bending portion of the push-in piece 108 has a tapered shape wherein the base end surface slopes toward the proximal end as it goes toward the left-right outside, whereby thick-walled reinforcing portions 118 are provided at the corner portions to improve strength and durability. The distal end portion of the push-in piece 108 is shaped and sized to allow insertion into the notch 96 of the pusher guide 82 in the axial direction from the proximal end side.

The proximal end of the push-in piece 108 is a large-diameter thick-walled portion 120, since the outer circumferential surface of the proximal end thereof with an arc-shaped cross section has a larger diameter than that of the distal end of the push-in piece 108. The large-diameter thick-walled portion 120 of the push-in piece 108 is inserted in the proximal portion 70 of the outer needle hub 60, which has a larger inner diameter dimension, in a movable state toward the distal end. By providing the large-diameter thick-walled portion 120 at the proximal end of the push-in piece 108, the gap between the push-in piece 108 and the outer needle hub 60 is suppressed even at the proximal portion 70 of the outer needle hub 60 with a larger inner diameter dimension. Thus, for example, when the push-in piece 108 is pushed toward the distal end as described below, damage due to excessive deformation of the push-in piece 108 is avoided, or the like.

As shown in FIGS. 1 and 2, the insertion tube 106 of the pusher 84 is inserted in the pusher guide 82, and the step surface 116 formed at the proximal end of the tapered distal end section 110 of the insertion tube 106 is engaged with the detent projection 104 of the pusher guide 82 in the axial direction. This limits the amount of movement of the pusher 84 to the proximal side relative the pusher guide 82, and the engagement of the detent projection 104 with the step surface 116 prevents the pusher 84 from being removed from the pusher guide 82. Since the outer circumferential surface of the tapered distal end section 110 and the edge widening part 105, which is the proximal side surface of the detent projection 104, are each tapered, when the insertion tube 106 of the pusher 84 is inserted into the pusher guide 82, the step surface 116 of the pusher 84 can easily overcome the detent projection 104 of the pusher guide 82 from the proximal side to the distal side.

The guide-side flat surfaces 100, 100 including the projecting distal end surfaces of the detent projections 104 of the pusher guide 82 are overlapped on the pusher-side flat surfaces 114, 114 of the pusher 84 in the up-down direction, as shown in FIG. 4. This allows the amount of relative rotation of the pusher 84 and the pusher guide 82 around the central axis (needle axis) to be limited by the contact section constituted by the contact between the pusher-side flat surface 114, which is a first rotation regulating surface, and the guide-side flat surface 100, which is a second rotation regulating surface. Thus, in this practical embodiment, the rotation limiting mechanism limiting the amount of relative rotation of the pusher 84 and the pusher guide 82 is realized with a simple structure by the contact between the guide-side flat surface 100 and the pusher-side flat surface 114.

The circumferential rotation of the pusher 84 relative to the pusher guide 82 is limited by the rotation limiting mechanism, and the circumferential rotation of the pusher guide 82 relative to the outer needle hub 60 is limited. As a result, the circumferential rotation of the pusher 84 relative to the outer needle hub 60 is limited. Accordingly, the pusher 84 and the pusher guide 82 are housed inside the outer needle hub 60, as positioned in a predetermined orientation relative to the outer needle hub 60 in the circumferential direction.

The guide-side flat surface 100 of the pusher guide 82 and the pusher-side flat surface 114 of the pusher 84 can be easily formed during the molding of the pusher guide 82 and the pusher 84 without any problems such as interference with the parting, and the rotation limiting mechanism can be easily obtained. In addition, by configuring the rotation limiting mechanism by the contact between the surfaces, it is easy to ensure strength and durability against contact pressure.

The inner needle unit 12 and the outer needle unit 14 are mutually assembled in a state wherein the inner needle 16 is inserted in the outer needle 58 to constitute the needle assembly 10, as shown in FIGS. 1 and 2. The inner needle 16 is inserted into the outer needle hub 60 through the proximal end side opening and then it is inserted into the outer needle 58 through the cut 86 in the valve body 80. The connection section 50 of the inner needle hub 18, including the protector rotation restriction part 52, is inserted in the proximal end opening of the outer needle hub 60, so that the inner needle hub 18 and the outer needle hub 60 are positioned relative to each other to some extent and the inner needle hub 18 is movable to the proximal side relative to the outer needle hub 60.

As also shown in FIG. 3, in the needle assembly 10, the circumferential positioning part 54 of the inner needle hub 18 is inserted in the positioning groove 76 provided in the flanged portion 74 of the outer needle hub 60. By so doing, the amount of relative rotation of the inner needle hub 18 and the outer needle hub 60 is limited by the contact between the circumferential positioning part 54 and the flanged portion 74. Also, since the amount of rotation of the needle tip protector 22 relative to the inner needle hub 18 is limited by contact with the protector rotation restriction part 52, the rotation of the needle tip protector 22 relative to the outer needle hub 60 is limited, so that the needle tip protector 22 is positioned relative to the outer needle hub 60 in the circumferential direction. Accordingly, in the needle assembly 10, the needle tip protector 22, the pusher guide 82, and the pusher 84, which are restricted in rotation relative to the outer needle hub 60, are limited in rotation relative to each other and mutually positioned in the circumferential direction.

In the needle assembly 10, the needle tip protector 22 attached to the inner needle 16 is inserted in the lumen of the outer needle hub 60 and positioned in the outer needle hub 60, as positioned on the proximal side of the pusher 84, as shown in FIGS. 1 and 2. More specifically, the needle tip protector 22 is arranged in the lumen of the outer needle hub 60 between the pairs of push-in pieces 108, 108 of the pusher 84 facing each other, as shown in FIG. 2. In other words, the insertion tube 106 of the pusher 84 is located on the distal side of the needle tip protector 22, and the pair of push-in pieces 108, 108 of the pusher 84 extend out toward the proximal end beside the needle tip protector 22, on both sides in the width direction.

Thus, the proximal end portion of the pusher 84 is shaped to branch away, comprising the pair of push-in pieces 108, 108 facing each other, and the needle tip protector 22 is inserted between the pair of push-in pieces 108, 108. This can shorten the length of the outer needle hub 60 compared to when the needle tip protector 22 and the pusher 84 are arranged in series without overlapping each other as viewed in the axis-perpendicular direction.

The pusher 84 is not arranged on both sides in the facing direction of the first and second side plate parts 26, 28 (the up-down direction) in the needle tip protector 22, and the first and second side plate parts 26, 28 are exposed to the radial outside via the openings between the pair of push-in pieces 108, 108 in the circumferential direction. This decreases the diameter of the outer needle hub 60 compared to when a part of the pusher is arranged on the upper and lower sides of the needle tip protector 22, and also can ensure an appropriate stroke of the approaching displacement of the first and second side plate parts 26, 28 when the needle tip protector 22 is operated as described below.

The needle tip protector 22 projects to the outside further than the pair of push-in pieces 108, 108 in the axis-perpendicular direction (the up-down direction), which is substantially orthogonal to the facing direction of the pair of push-in pieces 108, 108 (the left-right direction) (see FIG. 1). The first needle tip protection part 34 and the second needle tip protection part 44 of the needle tip protector 22 are inserted in the protector engagement groove 72 formed on the inner surface of the outer needle hub 60, and the resistance force against movement of the needle tip protector 22 to the proximal side relative to the outer needle hub 60 is exerted by contact between the first needle tip protection part 34 and the second needle tip protection part 44 and the inner surface of the protector engagement groove 72. The present invention is not limited to the above-described mode wherein the resistance force against the movement of the needle tip protector 22 to the proximal side relative to the outer needle hub 60 is exerted by the direct contact of the needle tip protector 22 with the outer needle hub 60. Alternatively, the resistance force may be exhibited by indirect contact of the needle tip protector 22 with the outer needle hub 60 via another member. Specifically, for example, the resistance force may be exerted by the needle tip protector 22 touching the pusher 84 or the pusher guide 82 whose movement to the proximal side relative to the outer needle hub 60 is restricted.

The first and second projection pieces 40, 48 of the needle tip protector 22 are arranged in close proximity to the inner surfaces of the pair of push-in pieces 108, 108 of the pusher 84 which face each other. The facing surfaces of the first and second projection pieces 40, 48 and the push-in pieces 108, 108 are all flat in this practical embodiment, and the contact of the facing surfaces limits the amount of relative rotation of the needle tip protector 22 and the pusher 84. Since the amount of rotation of the pusher 84 relative to the outer needle hub 60 is limited via the pusher guide 82, the amount of rotation of the needle tip protector 22 relative to the outer needle hub 60 is limited via the pusher 84 and the pusher guide 82.

In the needle assembly 10 constructed as described above, the inner needle 16 and the outer needle 58 puncture a blood vessel. When puncturing the blood vessel, blood flows into the lumen of the inner needle hub 18 through the lumen of the inner needle 16. Therefore, for example, by making the inner needle hub 18 transparent or translucent so that the interior can be seen, the flow of blood into the inner needle hub 18 can confirm the inner needle 16 puncturing the vessel.

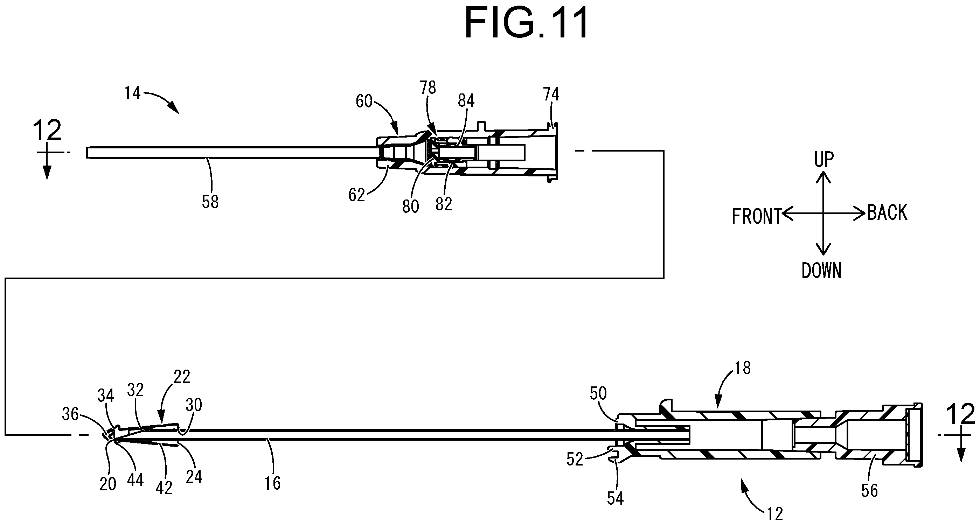

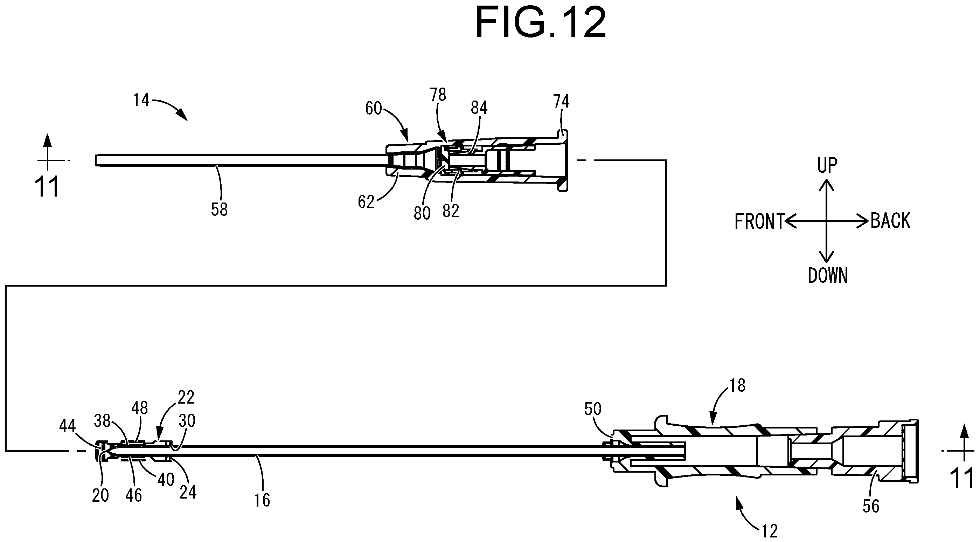

After the blood vessel is punctured with the inner needle 16 and the outer needle 58, the inner needle 16 is pulled out from the outer needle 58 to the proximal side, by pulling the inner needle unit 12 to the proximal side while holding the outer needle unit 14 in the puncture position. At this time, the connection between the inner needle hub 18 and the outer needle hub 60 is cancelled, and the entire inner needle unit 12 is separated from the outer needle unit 14, as shown in FIGS. 11 and 12. The outer needle unit 14 separated from the inner needle unit 12 stays in a state of puncturing the blood vessel. The inner needle 16 is pulled out from the cut 86 of the valve body 80, which seals the cut 86 of the valve body 80 in a liquid-tight manner, so that the lumen of the outer needle hub 60 is blocked by the valve body 80 and the blood flowing into the outer needle hub 60 through the outer needle 58 seldom leaks out to the proximal side of the outer needle hub 60.

When the inner needle 16 moves to the proximal side relative to the outer needle hub 60, the needle tip protector 22 moves to the distal side of the inner needle 16, because the first needle tip protection part 34 and the second needle tip protection part 44 are limited in movement to the proximal side by the protector engagement groove 72 in the outer needle hub 60. When the inner needle 16 moves to the proximal side further than the first and second needle tip protection parts 34, 44 of the needle tip protector 22 to cancel the contact between the inner needle 16 and the first and second needle tip protection parts 34, 44, the first and second side plate parts 26, 28 move inward in the facing direction so that they approach each other. As a result, the first and second needle tip protection parts 34, 44 move to a position covering the tip side of the inner needle 16, so that the needle tip 20 of the inner needle 16 is protected by the first and second needle tip protection parts 34, 44, and re-exposure of the inner needle 16 to the tip side is prevented by the needle tip protector 22.

The approaching displacement of the first and second needle tip protection parts 34, 44 releases the engagement between the first and second needle tip protection parts 34, 44 and the protector engagement groove 72 of the outer needle hub 60, thereby allowing the needle tip protector 22 to move to the proximal side relative to the outer needle hub 60. Then, a partially expanded portion provided near the tip of the inner needle 16 is engaged with the periphery of the insertion hole 30 in the bottom part 24 of the needle tip protector 22 in the axial direction. Owing to this, the needle tip protector 22, which protects the needle tip 20 of the inner needle 16, moves to the proximal side, integrally with the inner needle 16, so as to separate from the outer needle hub 60. Accordingly, the inner needle unit 12 separated from the outer needle unit 14 is in a needle tip-protected state wherein the needle tip 20 of the inner needle 16 is housed in the needle tip protector 22, and defects such as accidental touching of the needle tip 20 are avoided to realize excellent safety. In such a state of protection of the needle tip 20 by the needle tip protector 22, the inner needle 16 is surrounded by the first and second main bodies 32, 42, the first and second lateral cover pieces 38, 46, and the first and second projection pieces 40, 48 of the needle tip protector 22, and exposure of the needle tip 20 due to tilting of the inner needle 16 or the like is prevented, for example. Moreover, the lateral openings of the first needle tip protection part 34 are covered by the lateral guarding pieces 36, 36, so that exposure of the needle tip 20 is more effectively prevented.

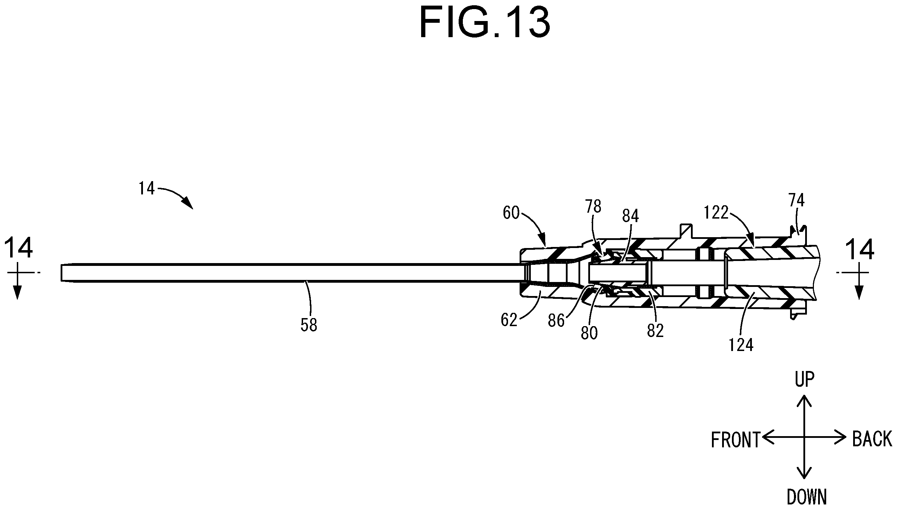

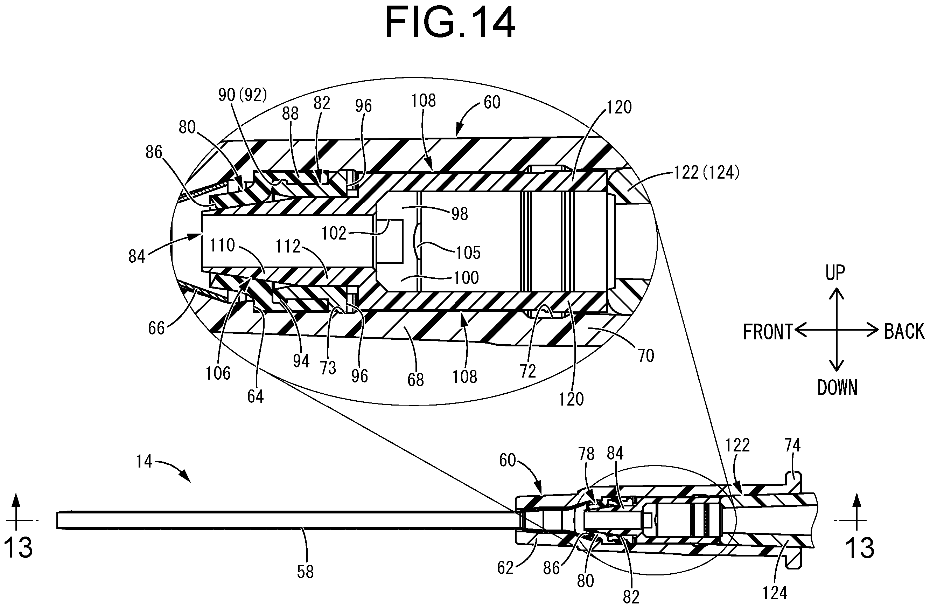

The outer needle unit 14, separated from the inner needle unit 12, stays as an indwelling needle in a state of puncturing the blood vessel, and an external circuit 122 is connected to the proximal side of the outer needle hub 60, as shown in FIGS. 13 and 14. The external circuit 122 is composed of, for example, a tube or a syringe, etc. In the drawings, a male connector 124 is shown, which constitutes the connection end of the external circuit 122 to the outer needle hub 60. The male connector 124 has a luer taper set on its outer circumferential surface corresponding to the inner surface of the proximal portion 70 of the outer needle hub 60, and can be inserted and fitted into the proximal portion 70. The connection end of the external circuit 122 to the outer needle hub 60 may have a locking portion that screws with the flanged portion 74 of the outer needle hub 60.

When the male connector 124 is inserted into the outer needle hub 60 from the proximal side, the distal end of the male connector 124 is pressed against the pair of push-in pieces 108, 108 of the pusher 84, to push the pusher 84 into the distal side. The pusher 84 moves toward the tip while being guided in the axial direction by the insertion tube 106 sliding on the inner surface of the pusher guide 82 including the groove-shaped recess 102. The pusher 84 is also guided in the axial direction by sliding contact between the pusher-side flat surfaces 114, 114 provided on the upper and lower sides of the pusher 84 and the guide-side flat surfaces 100, 100 provided on the upper and lower sides of the pusher guide 82.

In this practical embodiment, the pair of push-in pieces 108, 108 of the pusher 84 are provided extending out to the proximal side at the side of the needle tip protector 22. By so doing, it is possible that the protector engagement groove 72 of the outer needle hub 60, which is the engagement member with the first and second needle tip protection parts 34, 44 of the needle tip protector 22, is provided on the distal side, while on connecting the external circuit 122 to the outer needle unit 14, the pusher 84 is configured to be pushed in sufficiently to the distal side by the external circuit 122 (the male connector 124). As a result, for example, needle tip protection by the needle tip protector 22 is stably realized and reliability about opening operation of the valve mechanism is ensured.

The proximal end of the push-in piece 108, against which the male connector 124 is pressed, is the large-diameter thick-walled portion 120 to increase the contact area with the male connector 124 and ensure strength (durability) against contact. Additionally, the proximal end of the push-in piece 108, which is inserted into the proximal portion 70 of the outer needle hub 60 with a large inner diameter dimension, is the large-diameter thick-walled portion 120 with a large outer diameter dimension. This reduces the gap between the push-in piece 108 and the outer needle hub 60 in the radial direction and thus limits the amount of flexural deformation when the push-in piece 108 is pushed in, thereby avoiding damages, etc. to the push-in piece 108 when the push-in piece 108 is pushed in.

As the pusher 84 moves toward the tip, the insertion tube 106 of the pusher 84 is inserted into the cut 86 of the valve body 80 while pushing the cut 86 open. As a result, the distal end opening of the outer needle 58 and the external circuit 122 are connected through the lumen of the insertion tube 106. Thus, the lumen of the outer needle hub 60 is blocked by the valve body 80 before the connection of the external circuit 122, and it is switched to the connected state when the external circuit 122 is connected and the cut 86 is pushed open with the pusher 84. When the blockage of the internal flow path of the outer needle unit 14 by the valve body 80 is released, the outer needle unit 14 functions as an access port to the blood vessel. For example, the outer needle unit 14 is used as an administration port for fluid transfusion or liquid medicine, a blood collection port or a blood return port for artificial dialysis, and the like. In the insertion tube 106 of the pusher 84, the outer circumferential surface of the tapered distal end section 110 has a tapered shape whose diameter decreases as it goes toward the distal end so as to facilitate insertion into the cut 86 of the valve body 80.

In moving the pusher 84 to the distal side, the interference between the pair of push-in pieces 108, 108 of the pusher 84, which project on the both left-right sides, and the pusher guide 82 is avoided, and a sufficient travel stroke of the pusher 84 to the distal side is secured. In other words, the pair of push-in pieces 108, 108 of the pusher 84 are inserted into the pair of notches 96, 96 formed in the pusher guide 82, whereby the pair of push-in pieces 108, 108 can move to the distal side without interference with the pusher guide 82.

As the amount of circumferential relative rotation of the pusher 84 and the pusher guide 82 is limited by the rotation limiting mechanism, the pair of push-in pieces 108, 108 of the pusher 84 are positioned to be inserted into the pair of notches 96, 96 of the pusher guide 82. This positioning of the pair of push-in pieces 108, 108 of the pusher 84 relative to the pair of notches 96, 96 of the pusher guide 82 ensures the reliability of the opening operation of the valve mechanism by the movement of the pusher 84 toward the distal side, and also allows the movement of the pusher 84 to the distal side relative to the pusher guide 82 with a large travel stroke.

Moreover, the pair of guide pieces 98, 98 of the pusher guide 82 also guide the axial movement of the pusher 84, so that the pusher 84 can be guided in the axial direction by the pusher guide 82 while securing a large travel stroke of the pusher 84 to the distal side.

Although the practical embodiments of the present invention have been described in detail above, this invention is not limited by that specific description. For example, the rotation limiting mechanism is not necessarily limited to those configured by the contact of mutually overlapped flat surfaces. Alternatively, the rotation limiting mechanism can be configured by engagement of protrusions with each other or engagement of a convex portion with an inner surface of a concave portion.

When the contact section constituting the rotation limiting mechanism is composed of the first rotation regulating surface on the pusher 84 side and the second rotation regulating surface on the pusher guide 82 side, those first and second rotation regulating surfaces are not limited to flat surfaces such as the guide-side flat surface 100 and the pusher-side flat surface 114 shown in the aforesaid practical embodiment. That is, the contact section can be configured by curved surfaces, for example, as long as the relative rotation of the pusher 84 and the pusher guide 82 can be restricted by the mutual contact between them. Specifically, if the curvature of the outer circumferential surface of the pusher 84 and the curvature of the inner surface of the pusher guide 82 are smaller in the first and second rotation regulating surfaces than the curvature of the circle around the central axis, the relative rotation of the pusher 84 and the pusher guide 82 can be limited by the contact of the curved surfaces. In short, the first rotation regulating surface and the second rotation regulating surface can be configured by partially reducing the curvature of each of the outer circumferential surface of the pusher 84 and the inner surface of the pusher guide 82, which are overlapped on each other, in the circumferential direction, for example.

About the push-in piece 108, the mode of the pair of push-in pieces 108 arranged opposite each other as in the previous practical embodiment is preferable. However, for example, only a single push-in piece 108 may be provided, or three or more may be provided as separate from each other in the circumferential direction. Similarly, for the guide piece 98 of the pusher guide 82, only a single guide piece 98 may be provided, or three or more may be provided as separate from each other in the circumferential direction. The number of push-in pieces 108 and the number of guide pieces 98 are preferably the same, but may differ from each other.

The specific structure of the protector rotation restriction part 52 that restricts rotation of the needle tip protector 22 is not particularly limited. Specifically, for example, a convex protector rotation restriction part protruding from the distal end of the inner needle hub 18 may be inserted into a recess or hole formed in the bottom part 24 of the needle tip protector 22, thereby restricting rotation of the needle tip protector 22 relative to the inner needle hub 18. The rotation-limiting action may also be more effectively exerted by providing the protector rotation restriction part 52, which is overlapped on the side surface of the needle tip protector 22 as shown in the previous practical embodiment, at multiple locations in the circumferential direction.

The structure of the needle tip protector 22 is not limited to the specific example shown in the above-mentioned practical embodiment. That is, for example, it is also possible to employ a needle tip protector that has a protector body arranged radially outside the inner needle 16 and urged in such a direction that it is pressed against the outer circumferential surface of the inner needle 16 by magnetic force or the like, wherein the protector body moves to the position covering the tip side of the inner needle 16 upon withdrawal of the inner needle 16. In this case, the push-in piece 108 of the pusher 84 is arranged to extend toward the proximal end on the side of a protector housing that holds the protector body. Also, it is possible that a flexible piece extending from the protector housing that holds the protector body is arranged to pass through the side of the pusher to be engaged in a groove or the like on the inner surface of the outer needle hub. The needle tip protector is not limited to those covering only the tip portion of the inner needle 16, but may also cover the entire inner needle 16 in a containing state.

The insertion area of the pusher guide 82 into which the push-in piece 108 is inserted when the pusher 84 moves to the distal side is not necessarily limited to the notch 96 that penetrates the pusher guide 82 in the diametrical direction, but it may, for example, have a groove shape opening in the inner surface of the pusher guide 82. When a plurality of insertion areas are provided, they may be of different structures from each other. For example, at least one of them can be the notch 96 and at least one of them can be groove-shaped.

In the above-mentioned practical embodiment, there is shown an example wherein the pusher guide 82 is a separate member from the outer needle hub 60 and is attached to the outer needle hub 60. Alternatively, the pusher guide can also be configured by the outer needle hub, for example, by comprising two parts and setting the function of guiding the pusher 84 in one of those two parts. In the aforesaid practical embodiment, the pusher guide 82 has the function of guiding the pusher 84, as well as a function as a valve holding member to hold the valve body 80. However, for example, an insertion area can be provided for the valve holding member that does not have the function of guiding the pusher 84, into which the push-in piece 108 is inserted when the pusher 84 advances.

### KEYS TO SYMBOLS

10: Needle assembly; 12: Inner needle unit; 14: Outer needle unit; 16: Inner needle; 18: Inner needle hub; 20: Needle tip; 22: Needle tip protector; 24: Bottom part; 26: First side plate part; 28: Second side plate part; 30: Insertion hole; 32: First main body; 34: First needle tip protection part; 36: Lateral guarding piece; 38: First lateral cover piece; 40: First projection piece; 42: Second main body; 44: Second needle tip protection part; 46: Second lateral cover piece; 48: Second projection piece; 50: Connection section; 52: Protector rotation restriction part; 54: Circumferential positioning part; 56: Filter cap; 58: Outer needle; 60: Outer needle hub; 62: Peripheral wall; 64: Step; 66: Swaging pin; 68: Distal portion; 70: Proximal portion; 72: Protector engagement groove; 73: Concave groove; 74: Flanged portion; 76: Positioning groove; 78: Hemostasis valve mechanism; 80: Valve body; 82: Pusher guide; 84: Pusher; 86: Cut (insertion part); 88: Support piece; 90: Retaining groove; 92: Widened portion; 94: Outer circumferential projection; 96: Notch; 98: Guide piece; 100: Guide-side flat surface (second rotation regulating surface); 102: Groove-shaped recess; 104: Detent projection; 105: Edge widening part; 106: Insertion tube; 108: Push-in piece; 110: Tapered distal end section; 112: Flat proximal end section; 114: Pusher-side flat surface (first rotation regulating surface); 116: Step surface; 118: Reinforcing portion; 120: Large-diameter thick-walled portion; 122: External circuit; 124: Male connector

## Claims

1. A needle assembly comprising:
an inner needle with an inner needle hub at its proximal side;
a hollow outer needle with an outer needle hub at its proximal side;
a needle tip protector protecting a needle tip of the inner needle removed from the outer needle;
a valve body blocking a lumen of the outer needle hub;
a pusher pushing a cut of the valve body open by moving toward the needle tip of the inner needle so as to release blockage of the outer needle hub by the valve body, the pusher including a push-in piece extending toward a proximal end beside the needle tip protector; and
a tubular pusher guide guiding movement of the pusher toward the needle tip, the pusher guide including an insertion area which allows insertion of the push-in piece therein.

2. The needle assembly according to claim 1, wherein a notch penetrating a circumferential wall of the tubular pusher guide, while opening an inside to an outside, is provided as the insertion area.

3. The needle assembly according to claim 1 or 2, further comprising a protector rotation restriction part provided at a distal end of the inner needle hub and inserted between the needle tip protector and the outer needle hub so as to restrict rotation of the needle tip protector relative to the inner needle hub by contacting the needle tip protector.

4. A needle assembly comprising:
an inner needle with an inner needle hub at its proximal side;
a hollow outer needle with an outer needle hub at its proximal side;
a needle tip protector protecting a needle tip of the inner needle removed from the outer needle;
a valve body blocking a lumen of the outer needle hub;
a pusher pushing a cut of the valve body open by moving toward the needle tip so as to release blockage of the outer needle hub by the valve body, the pusher including a push-in piece extending toward a proximal end beside the needle tip protector;
a tubular pusher guide attached to the outer needle hub to guide movement of the pusher toward the needle tip; and
a rotation limiting mechanism limiting rotation of the pusher relative to the pusher guide assembled and retained in the outer needle hub.

5. The needle assembly according to claim 4, wherein the rotation limiting mechanism is constituted by a contact section between the pusher and the pusher guide so as to limit relative rotation of the pusher and the pusher guide in a circumferential direction by mutual contact.

6. The needle assembly according to claim 5, wherein
a proximal end of a tapered distal end section of the pusher is provided with a step surface, and a cross section of the proximal end of the tapered distal end section of the pusher is not a true circle, and the pusher guide is provided with a detent projection to prevent removal of the pusher by contacting the step surface, and
the contact section is constituted by a projecting distal end surface of the detent projection of the pusher guide and an outer peripheral surface of the pusher.

7. The needle assembly according to any one of claims 1-6, wherein
an inner diameter dimension of the outer needle hub is larger in a proximal portion than in a distal portion, and
a part of the push-in piece of the pusher that is inserted into the proximal portion of the outer needle hub has a large-diameter thick-walled portion with a large outer diameter dimension and a thick wall.

8. The needle assembly according to any one of claims 1-7, wherein a radially inner edge of a proximal end of the pusher guide is provided with an edge widening part that slopes radially outward as it goes toward the proximal end.

9. A needle assembly comprising:
an inner needle with an inner needle hub at its proximal side;
a hollow outer needle with an outer needle hub at its proximal side;
a needle tip protector being positioned in the outer needle hub and protecting a needle tip of the inner needle removed from the outer needle; and
a protector rotation restriction part provided in the inner needle hub so as to restrict rotation of the needle tip protector relative to the inner needle hub.

10. A needle assembly comprising:
an inner needle with an inner needle hub at its proximal side;
a hollow outer needle with an outer needle hub at its proximal side;
a needle tip protector protecting a needle tip of the inner needle removed from the outer needle;
a valve body blocking a lumen of the outer needle hub;
a pusher pushing a cut of the valve body open by moving toward the needle tip of the inner needle so as to release blockage of the outer needle hub by the valve body, the pusher including a push-in piece extending toward a proximal end beside the needle tip protector; and
a valve holding member holding the valve body in an attached state to the outer needle hub, the valve holding member including an insertion area which allows insertion of the push-in piece therein.

11. A needle assembly comprising:
an inner needle with an inner needle hub at its proximal side;
a hollow outer needle with an outer needle hub at its proximal side;
a valve body blocking a lumen of the outer needle hub;
a pusher pushing a cut of the valve body open by moving toward a needle tip of the inner needle so as to release blockage of the outer needle hub by the valve body; and
a tubular pusher guide guiding movement of the pusher toward the needle tip, the pusher guide including an insertion area into which the pusher is inserted therein partially in a circumferential direction by the movement of the pusher toward the needle tip.
